# EUROPEAN PATENT APPLICATION

(11) **EP 3 974 536 A1**
(43) Date of publication of application: **30.03.2022**
(21) Application number: 21823461.5
(22) Date of filing: 30.07.2021
(51) Int. Cl.: C12Q 1/04, C12Q 1/68, G16H 20/10

(54) **BACTERIUM PROVISION SYSTEM**

(30) Priority: 30.07.2020 JP 2020129676
(71) Applicant: Noster Inc., Muko-shi, Kyoto 617-0006 (JP)
(72) Inventor: KITAO, Kohey, Muko-shi, Kyoto 6170006 (JP)
(74) Representative: J A Kemp LLP
(86) International application number: PCT/JP2021/028233
(87) International publication number: WO 2022/025222

(57) **Abstract**

A system that stores bacterial flora of donors, selects a bacterium necessary for improving bacterial flora according to the health condition of a test subject, and supplies the same in a utilizable state to the test subject is provided.

## Description

### [Technical Field]

The present invention relates to a system that stores bacterial flora derived from biological samples including feces, and supplies bacteria contained in the bacterial flora when necessary.

### [Background Art]

Bacterial flora means the whole microorganism that exists in a certain place. The types of bacteria that constitute the bacterial flora vary depending on the individual and the sites where they exist, and bacterial flora is subject to change depending on the lifestyle and what is eaten or drunk. In addition, bacterial flora changes in response to the health condition of individual, and it has been reported that the intestinal bacterial flora of patients with cardiovascular disease actually contains less Bacteroides vulgatus and less Bacteroides dorei. Research is ongoing to prevent or treat cardiovascular diseases by amplifying these bacteria (patent document 1). In recent years, research on bacterial flora in various body parts has been conducted, and in particular, drug discovery research using the intestinal bacterial flora has been conducted.

For example, as regards useful bacteria such as bifidobacteria and the like, attempts have been made to increase the number of useful bacteria by ex vivo growth of a strain detected in the feces of a test subject and transplantation of the same to the test subject. However, the intestinal bacterial flora also contains bacteria that are difficult to culture, and useful effects of them are currently under investigation.

In addition, as a treatment method for patients with intestinal dysbiosis, a method of transplanting feces from another person is being studied. Current fecal transplantation aims to improve symptoms of a patient with disbiosis by transplanting the feces of another healthy person into the patient. However, since the feces of the patient in good health is not transplanted, colonization of the bacterial flora poses a problem. In the case of administering intestinal bacteria, administration of the originally existing bacteria is considered to be more advantageous in terms of colonization. As to the bacteria required for each individual, therefore, it is necessary to take flexible measures based on, for example, whether it is better to take advantage of other's bacteria, or the person's own bacteria are to be utilized. At present, there is no system for supplying such bacteria.

### [Document List]

### [Patent document]

patent document 1: WO 2019/156251

### [Summary of Invention]

### [Technical Problem]

The problem in the present invention is to construct a system that selects a bacterium necessary for improving the health condition of a test subject, and supplies the same to the test subject in a providable state.

### [Solution to Problem]

The present inventors have conducted intensive studies in view of the above-mentioned problem. As a result, they have constructed a database containing data in which the changes in the health condition of the donors of the bacterial flora are associated with the changes in the provided bacterial flora, and a bacterial flora bank in which the provided bacterial flora is stored, selected a bacterium necessary for the recovery of the health condition of a test subject based on the changes in the health condition of the test subject by using the above-mentioned database, and provided the bacterium selected from the bacterial flora bank to the test subject, thus completing a system that supplies a bacterium to a test subject.

Accordingly, the present invention provides the following.
[1] A system that supplies a bacterium to a test subject, comprising
   (A) a step of constructing a database comprising data in which the changes in the health condition of donors of bacterial flora are mutually associated with the changes in the provided bacterial flora, and a bacterial flora bank in which the provided bacterial flora is stored,
   (B) a step of selecting a bacterium necessary for the recovery of the health condition of a test subject based on the changes in the health condition of the test subject by using the above-mentioned database,
   (C) a step of supplying the bacterium selected from the bacterial flora bank to the test subject.
[2] The system of [1], wherein the step (A) comprises the following steps:
   (A-1) a step of analyzing the health condition of the donors of the bacterial flora and the provided bacterial flora, and constructing a database comprising data in which the changes in the health condition of the donors of the bacterial flora are mutually associated with the changes in the provided bacterial flora,
   (A-2) a step of storing the provided bacterial flora and constructing a bacterial flora bank.
[3] The system of [2], wherein the step (A) further comprises the following step:
   (A-3) a step of updating the database and the bacterial flora bank by repeating steps (A-1) and (A-2).
[4] The system of any one of [1] to [3], wherein the step (B) comprises the following steps:
   (B-1) a step of analyzing the health condition of the test subject and the bacterial flora of the test subject, and recording data in which the changes in the health condition of the test subject are mutually associated with the changes in the bacterial flora of the test subject on the database in step (A) ,
   (B-2) a step of storing the bacterial flora of the test subject in the bacterial flora bank of step (A),
   (B-3) a step of searching the database for changes in the health condition of the donor which are the same as or similar to the changes in the health condition of the test subject,
   (B-4) a step of confirming changes in the bacterial flora of the donor associated with the obtained changes in the health condition of the donor,
   (B-5) a step of selecting a bacterium necessary for the recovery of the health condition of the test subject.
[5] The system of any one of [1] to [4], wherein the step (C) comprises the following steps:
   (C-1) a step of isolating a bacterium selected from the bacterial flora bank,
   (C-2) a step of proliferating the isolated bacterium,
   (C-3) a step of supplying the proliferated bacterium to the test subject.
[6] The system of any one of [1] to [5], wherein the bacterial flora is a bacterial flora selected from the group consisting of intestinal bacterial flora, oral bacterial flora, skin bacterial flora, and vaginal bacterial flora.
[7] The system of any one of [1] to [6], wherein the analysis of the bacterial flora comprises identification of bacteria contained in the bacterial flora and measurement of an abundance ratio thereof.
[8] The system of [7], wherein the analysis of the bacterial flora is 16s metagenome analysis or 23s metagenome analysis.
[9] The system of [8], comprising a sequencer for the 16s metagenome analysis or the 23s metagenome analysis.
[10] The system of any one of [1] to [9], wherein the health condition is a condition of infection and parasitic disease, tumor, endocrine and metabolism disease, psychiatric and nerve system disease, circulatory disease, respiratory disease, gastrointestinal disease, skin and subcutaneous tissue disease, renal genitourinary tract disease, autoimmune disease, or collagen disease.
[11] The system of any one of [1] to [10], comprising a non-volatile storage memory for storing database.

### [Advantageous Effects of Invention]

According to the present invention, by constructing a bacterial flora bank and a database containing data in which changes in the health condition of a test subject and donors of bacterial flora are associated with the changes of the bacterial flora, a bacterium necessary for improving the health condition of the test subject can be appropriately selected, based on the database, from the bacterial flora of the test subject himself or herself or the donors of the bacterial flora which are contained in the bacterial flora bank, based on the database, and supplied to the test subject in a providable state. As a result, the health condition of the test subject can be improved.

### [Brief Description of Drawings]

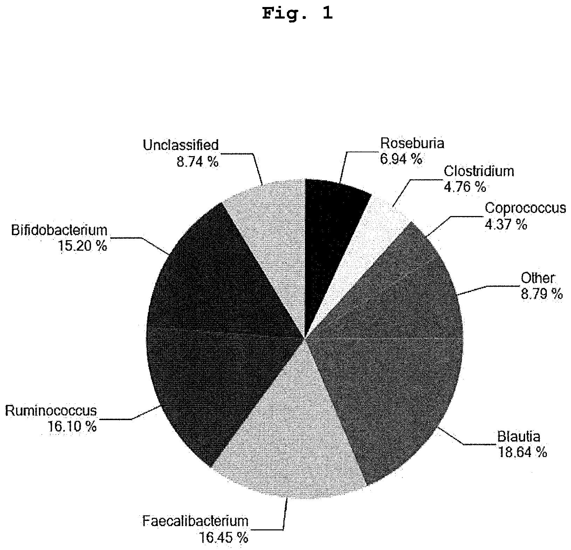

Fig. 1 shows an example of the results of 16S metagenome analysis of bacterial flora (feces).

### [Description of Embodiments]

The present invention is explained in detail in the following.

The present invention provides a system that supplies a bacterium to a test subject (the system of the present invention).

The test subject to which the system of the present invention can be applied is not particularly limited as long as bacterial flora can be recovered from the test subject. Examples of the test subject include humans and mammals (e.g., monkeys, bovines, horses, swine, mice, rats, guinea pigs, hamsters, dogs, cats, rabbits, sheep, goats, etc.) other than humans. A preferred test subject is a human.

The bacterium that can be supplied by the system of the present invention is not particularly limited as long as it constitutes the bacterial flora harvested from a test subject and can be proliferated ex vivo. Examples of such bacterium include Bacteroides, Parabacteroides, Alistipes, Dorea, Ruminococcus, Faecalibacterium, Collinsella, Roseburia, Coprococcus, Subdoligranulum, Holdemania, Butyrivibrio, Anaerotruncus, Blautia, Fusobacterium, Leptotrichia, Vibrio, Lactobacillus, Eubacterium, Propionibacterium, Bifidobacterium, Actinomyces, Clostridium, Peptococcus, Peptostreptococcus, Veillonella, Spirochaetes, Staphylococcus, Streptococcus, Enterococcus, Neisseria, Corynebacterium, Mycobacterium, Klebsiella, Proteus, Pseudomonas, Haemophilus, Mycoplasma, Prevotella, Gemella, Rothia, Granulicatella, Peptoniphilus, Delftia, Escherichia, Gardnerella, Mobiluncus, Finegoldia, Micromonas, Anaerococcus, Atopobium, Ureaplasma and the like.

The system of the present invention includes the following steps:
(A) a step of constructing a database comprising data in which the changes in the health condition of donors of bacterial flora are mutually associated with the changes in the provided bacterial flora, and a bacterial flora bank in which the provided bacterial flora is stored
(B) a step of selecting a bacterium necessary for the recovery of the health condition of a test subject based on the changes in the health condition of the test subject by using the above-mentioned database
(C) a step of supplying the bacterium selected from the bacterial flora bank to the test subject.

The system of the present invention includes a step of constructing a database comprising data in which the changes in the health condition of donors of the bacterial flora are mutually associated with the changes in the provided bacterial flora, and a bacterial flora bank in which the provided bacterial flora is stored (step (A)).

The step (A) may also include the following steps.
(A-1) a step of analyzing the health condition of the donors of the bacterial floras and the provided bacterial flora, and constructing a database comprising data in which the changes in the health condition of the donors of the bacterial floras are mutually associated with the changes in the provided bacterial flora.
(A-2) a step of storing the provided bacterial flora and constructing a bacterial flora bank.

In step (A), the donors of bacterial flora are not particularly limited as long as bacterial flora can be recovered from the donors, as in the test subject to which the system of the present invention can be applied. Examples of the donor include humans and mammals (e.g., monkeys, bovines, horses, swine, mice, rats, guinea pigs, hamsters, dogs, cats, rabbits, sheep, goats, etc.) other than humans. Preferred donors are humans.

The number of the donors of the bacterial floras is not particularly limited as long as it guarantees the level where the system of the present invention can be practiced with high accuracy. It is generally not less than 300, preferably not less than 500, more preferably not less than 1,000, and most preferably not less than 10,000.

In step (A), the changes in the health condition of the donors of the bacterial floras can be grasped by comparing respective health conditions at respective times of donation by the donors. Specifically, respective health conditions can be compared by analyzing respective health conditions and comparing the results thereof.

The analysis of respective health conditions is not particularly limited as long as the results that afford comparison of respective health conditions can be obtained. For example, gender, age, height, weight, blood pressure, eyesight, audibility, blood test results, urine test results, and the like can be the analysis targets. Therefore, by measuring the above-mentioned items for the donor simultaneously with the collection of bacterial flora, the health condition of each donor at the time of donation can be grasped. Alternatively, as another method, the health condition of each donor at the time of donation can be grasped by conducting a questionnaire to the donor simultaneously with the collection of bacterial flora. Examples of the questionnaire items include gender, age, height, body weight, surgical history, previous disease, the presence or absence of disease during treatment, type of medications being taken, the presence or absence of smoking or drinking alcohol, and the like. Particularly, as the disease during treatment, infection and parasitic disease, tumor, endocrine and metabolism disease, psychiatric and nerve system disease, circulatory disease, respiratory disease, gastrointestinal disease, diseases of skin and subcutaneous tissue, renal genitourinary tract disease, autoimmune disease and collagen disease, diseases of eyes and ears, and the like can be mentioned.

Examples of the infection and parasitic disease include intestinal infections, tuberculosis, viral hepatitis, fungal disease, and the like.

Examples of the tumor include malignant tumor in the stomach, malignant tumor in the intestines, malignant tumor in the lungs, a trachea, and bronchi, malignant tumor in the liver tract (hepatic and biliary tract), malignant tumor in the breast, malignant tumor in the uterus, malignant lymphoma, leukemia, and benign tumor.

Examples of the endocrine and metabolism disease include thyroid gland disorder, diabetes, dyslipidemia, hyperuricemia, gout, osteoporosis, obesity, and the like.

Examples of the psychiatric and nerve system disease include dementia, schizophrenia, mood disorder (including bipolar disorder), neurotic disorder, Parkinson's disease, Alzheimer's disease, epilepsy, autonomic nerve system disorder, and the like.

Examples of the circulatory disease include hypertensive disease, ischemic cardiac disease, cardiac failure, arrhythmia, subarachnoid hemorrhage, intracerebral bleeding, cerebral infarction, cerebral arterial sclerosis, arteriosclerosis, aneurysm, phlebeurysm, hypotension, and the like.

Examples of the respiratory disease include nasopharyngitis, pharyngitis, tonsillitis, pneumonia, bronchitis, allergic rhinitis, chronic rhinosinusitis, chronic obstructive pulmonary disease, asthma, and the like.

Examples of the gastrointestinal disease include dental caries, gingival inflammation, periodontal disease, gastric ulcer, duodenal ulcer, gastritis, duodenal inflammation, polyp of the colon, irritable bowel syndrome, hemorrhoid, alcoholic hepatic diseases, fatty liver, chronic hepatitis (excluding alcoholic ones), cirrhosis (excluding alcoholic ones), cholelithiasis, cholecystitis, pancreatic disease, and the like.

Examples of the skin and subcutaneous tissue disease include infections of skin or subcutaneous tissue, dermatitis (including atopic dermatitis), eczema, and the like.

Examples of the renal genitourinary tract disease include glomerulus disease, renal tubule interstitial disease, renal failure, urinary tract stone disease, prostatomegaly, menstrual disorder and perimenopausal disorder, endometriosis, infertility (implantation disorder, recurrent pregnancy loss), and the like.

Examples of the autoimmune disease and collagen disease include rheumatoid arthritis, systemic lupus erythematosus, polymyositis, scleroderma, Sjogren's syndrome, Behcet's disease, myasthenia gravis, multiple sclerosis, autoimmune hepatitis, ulcerative colitis, Crohn's disease, psoriasis, graves disease, chronic thyroiditis, IgA nephropathy, circular shape alopecia, and the like.

Examples of the disease of eyes and ears include conjunctivitis, cataract, otitis externa, otitis media, Meniere's disease, and the like.

In addition, the subjective symptoms of donors can also be recited as items. For example, stiff neck, lumbago, numbness in a limb, dizziness, constipation, diarrhea, and the like can be mentioned.

Differences obtained as decribed above in the numerical values between analysis results at respective donation time points and in response results of questionnaire items can be shown as changes in the health condition of the donors of the bacterial flora.

In step (A), the changes in the provided bacterial flora can be grasped by analyzing respective bacterial floras provided by the donors at respective donation time points mentioned above and comparing the results thereof. Specifically, the comparison of respective bacterial floras can be performed by analyzing the bacteria contained in the bacterial floras and the abundance ratio thereof and comparing the results thereof.

Differences in the abundance ratios of the bacteria contained in the bacterial floras at respective donation time points obtained as described above can be shown as the changes in the bacterial flora.

The bacterial flora provided by the donors is not particularly limited as long as the entire microorganism provided from a specific site of the donor is alive. For example, an intestinal bacterial flora, an oral bacterial flora, a skin bacterial flora, a vaginal bacterial flora, and the like can be mentioned. The bacterial flora may be provided as any form of a sample depending on the site from which it is derived, and examples include mucosal or skin lavage fluid, feces, mucous membrane, saliva, sweat, secretions, and the like. The number of bacteria contained in the provided bacterial flora is not particularly limited as long as the bacteria can grow after storage. For example, it is generally 1.0×10⁴ - 1.0×10¹² cells/bacterial flora.

The bacteria contained in the bacterial flora and the abundance ratio thereof can be analyzed by a known method. Examples of the method include Gram's staining method, microscopy, PCT test method, antibody test method, and the like. Due to the advantage that the bacteria contained in the bacterial floras and the abundance ratio thereof can be simultaneously analyzed, PCR test method is preferable. Among others, as the method using real-time PCR, 16S metagenome analysis and 23S metagenome analysis can be mentioned, and 16S metagenome analysis is preferred. When 16S metagenome analysis is performed, the genome region to be analyzed is not particularly limited as long as the bacterium can be determined, the 16S rRNA gene centered on V3 to V4 region is preferably used.

When the bacteria contained in the bacterial flora and the abundance ratio thereof are analyzed using real-time PCR, a next-generation sequencer is preferably used since plural samples can be analyzed simultaneously. Next-generation sequencers can simultaneously determine the base sequences of tens to hundreds of millions of randomly cleaved DNA fragments in parallel. The next-generation sequencer used in the present invention is not particularly limited as long as it can simultaneously determine the base sequences of randomly cleaved DNA fragments in parallel. For example, MiSeq (Illumina, Inc.) can be mentioned. The data obtained by the next-generation sequencer can be used to identify the bacteria constituting the bacterial flora and determine the abundance ratio in the bacterial floras by using the reference genome database, Silva. Reference genome databases are not particularly limited as long as they can identify the bacteria constituting the bacterial flora.

Each time point of donation of the bacterial flora may be determined arbitrarily. For example, each time point of donation may be determined so that the period between the closest time points of donation will be constant. Examples of the constant period include generally 360 days, preferably 180 days, most preferably 90 days, and the like. Alternatively, the point when the health condition of donor changed may be set as each time point of donation.

The number of donations of the bacterial flora by the donors is not particularly limited as long as it is not less than two times. However, higher numbers of donations of the bacterial floras by the donors is preferable, because the system of the present invention can more accurately select the bacterium to be provided to a test subject by monitoring the changes in the health condition of the donors for a long period of time. Therefore, the number of donations of the bacterial flora by the donors is not less than two times, preferably not less than three times, more preferably not less than four times, most preferably not less than five times.

As described above, a database comprising data in which the changes in the health condition of the donors of the bacterial flora are mutually associated with the changes in the provided bacterial flora can be constructed. As used herein, that the changes in the health condition of the donors of the bacterial flora are mutually associated with the changes in the provided bacterial flora means that changes in the analysis items of the health condition of the donor of the bacterial flora are correlated with changes in the bacteria contained in the provided bacterial flora and the abundance ratio thereof. For example, changes in the analysis items of the health condition of the donor of the bacterial flora can be correlated with changes in the bacteria contained in the provided bacterial flora and the abundance ratio thereof before and after the onset of the above-mentioned diseases under treatment.

The data in which the changes in the health condition of the donors of the bacterial flora are mutually associated with the changes in the provided bacterial flora are used to construct the database. The database can be stored in non-volatile storage and recalled as necessary. Examples of the non-volatile storage include a non-volatile memory installed in a computer and a non-volatile memory independent of a computer. Examples of the non-volatile memory installed in a computer include a hard disk. Examples of the non-volatile memory independent of a computer include floppy disc, optical disc (CD, DVD, BD, and the like), and semiconductor memory (flash memory and the like).

In step (A), the storage method of the provided bacterial flora is not particularly limited as long as the entire microorganism contained in the bacterial flora of donor can grow. For example, cryopreservation can be mentioned. A specific method for cryopreservation includes a method in which a sample in any form containing bacterial flora is instantaneously frozen in liquid nitrogen directly or after a pre-treatment. Examples of the pre-treatment include a treatment by adding an antifreezing agent so that intracellular freezing will not occur. The antifreezing agent includes, for example, glycerol, dimethyl sulfoxide (DMSO), ethylene glycol, and the like. The frozen bacterial flora can be stored for a long period of time in a storage capable of storing at a low temperature (liquid nitrogen tank, ultra-low temperature freezer at -80°C, etc.) and can be grown again if necessary. Alternatively, as other storage methods, freeze-dry method, L-dry method, low temperature passage culture method, liquid paraffin overlay method, suspension storage method, soft agar method, and the like can be mentioned.

The bacterial flora bank constructed by the stored bacterial flora is not particularly limited as long as bacterial flora provided by different donors is stored for respective donors, and bacterial flora provided by the same donor is stored at each time point of donation. The number of bacterial flora populations stored for the construction of the bacterial flora bank is not particularly limited as long as it is 2 or more populations. However, a larger number of populations of the stored bacterial flora that construct the bacterial flora bank is preferable, since the system of the present invention can more accurately select the bacterium to be provided to a test subject. Therefore, the number of populations of the stored bacterial flora that construct the bacterial flora bank is not less than 2 populations, preferably not less than 5 populations, more preferably not less than 7 populations, and most preferably not less than 10 populations.

In addition, step (A) may further contain the following step:
(A-3) a step of updating the database and the bacterial flora bank by repeating step (A-1) and (A-2).

As mentioned above, a higher number of donations of the bacterial flora by the donors in the database is preferable and a larger number of populations of the stored bacterial flora that construct the bacterial flora bank is preferable. Therefore, in the system of the present invention, the database and the bacterial flora bank are preferably updated by repeating steps (A-1) and (A-2). The number of repeats of the steps (A-1) and (A-2) is not particularly limited, and is not less than two times, preferably not less than three times, more preferably not less than four times, and most preferably not less than five times.

The system of the present invention includes a step of selecting a bacterium necessary for the recovery of the health condition of a test subject based on the changes in the health condition of the test subject by using the above-mentioned database (step (B)).

In addition, step (B) may further contain the following steps:
(B-1) a step of analyzing the health condition of the test subject and the bacterial flora of the test subject, and recording data in which the changes in the health condition of the test subject and the changes in the bacterial flora of the test subject are mutually associated in the database of step (A)
(B-2) a step of storing the bacterial flora of the test subject in the bacterial flora bank of step (A)
(B-3) a step of searching the database for changes in the health condition of the donor which are the same as or similar to the changes in the health condition of the test subject
(B-4) a step of confirming the changes in the bacterial flora of the donor associated with the obtained changes in the health condition of the donor
(B-5) a step of selecting a bacterium necessary for the recovery of the health condition of the test subject.

In step (B), the analysis of the health condition of the test subject and grasp of the changes, analysis of the bacterial flora of the test subject and grasp of the changes, and associating the changes in the health condition of the test subject with the changes in the bacterial flora of the test subject may be performed by the same methods as those for the donors of bacterial flora in step (A). The data obtained from the test subjects are recorded in the database constructed in step (A), and the bacterial flora obtained in the test subjects is stored in the bacterial flora bank constructed in step (A). Therefore, the test subjects themselves may be included in the donors in step (B).

In step (B), changes in the health condition of the donor which are the same as or similar to the changes in the health condition of the test subject are searched for in the database. The changes in the health condition of the donor which are the same as or similar to the changes in the health condition of the test subject are searched for by referring to the changes in the health condition of the donors which are recorded in the database constructed in step (A), and selecting the changes in the health condition of the donors which are the same as or similar to the changes in the health condition of the test subject. To be specific, the search is conducted by calling, on a computer, the database in which the changes in the health condition of the donors (differences in the numerical values between analysis results at respective donation time points and differences in response results of questionnaire items) are recorded, and searching for the changes in the health condition of the donor which are the same as or similar to the changes in the health condition of the test subject (differences in the numerical values between analysis results at the respective donation time points and differences in response results of questionnaire items). As used herein, the similar changes in the health condition refer to the most similar changes in the health condition, even if the difference in the numerical values of the analysis results between the test subject and the donor and the difference in the response results of questionnaire items are not completely the same.

In step (B), changes in the bacterial flora of the donor associated with the obtained changes in the health condition of the donor are confirmed. The changes in the bacterial flora of the donor associated with the obtained changes in the health condition of the donor, which are obtained as a result of the search, are confirmed by referring to the changes in the bacterial flora of the donors which are recorded in the database constructed in step (A), and selecting the changes in the bacterial flora of the donor associated with the changes in the health condition of the donor obtained as a result of the search. To be specific, the changes are confirmed by calling, on a computer, the database in which the changes in the bacterial flora of the donors associated with the changes in the health condition of the donors obtained as a result of the search (difference in the abundance ratio of bacteria contained in the bacterial flora at respective donation time points) are recorded, and selecting the changes in the bacterial flora of the donor associated with the changes in the health condition of the donor obtained as a result of the search (difference in the abundance ratio of bacteria contained in the bacterial floras at the respective donation time points).

In step (B), a bacterium necessary for the recovery of the health condition of the test subject is selected. The necessary bacterium can be selected by confirming the changes in the abundance ratio of the bacteria contained in the associated and selected bacterial flora of a donor. To be specific, a bacterium that showed a decreased abundance ratio and a bacterium that showed an increased abundance ratio before and after the changes are specified. As for the bacterium that showed a decreased abundance ratio, the bacterium can be selected as the bacterium necessary for the recovery of the health condition of the test subject. On the other hand, as for the bacterium that showed an increased abundance ratio, bacteria other than the bacterium that showed an increased abundance ratio can be selected as the bacterium necessary for the recovery of the health condition of the test subject from all the bacteria contained in the bacterial flora.

The system of the present invention includes a step of supplying a bacterium selected from the bacterial flora bank to a test subject (step (C)).

Step (C) may further contain the following steps:
(C-1) a step of isolating the bacterium selected from the bacterial flora bank
(C-2) a step of growing the isolated bacterium
(C-3) a step of supplying the grown bacterium to a test subject.

In step (C), a bacterium selected from the bacterial flora bank is provided to a test subject. The bacterium selected in step (B) as a bacterium necessary for the recovery of the health condition of the test subject is a bacterium contained in the bacterial flora stored in the bacterial flora bank. Therefore, provision of the bacterium selected from the bacterial flora bank to the test subject can be performed by isolating the bacterium selected from the bacterial flora bank as a bacterium necessary for the recovery of the health condition of the test subject in step (B) from the bacterial flora bank and growing the bacterium.

The bacterium selected from the bacterial flora bank as a bacterium necessary for the recovery of the health condition of the test subject in step (B) can be isolated from the bacterial flora bank by a known means. The necessary bacterium is a bacterium contained in the bacterial flora of the donor associated with the changes in the health condition of the donor, which are obtained as a result of the search in step (B), among the bacterial flora stored in the bacterial flora bank. Therefore, the desired bacterium can be isolated by selecting a bacterial flora containing the bacterium from the bacterial flora bank, and separation culturing the bacterium. For example, the cryopreserved bacterial flora from the bacterial flora bank is grown in a liquid medium, and then smeared on a solid medium suitable for the growth of the desired bacterium. The solid medium is cultured under the conditions suitable for the growth of the desired bacterium, and the desired bacterium can be obtained formed a single colony. The solid medium and culture conditions suitable for the growth of the desired bacterium can be appropriately selected by those of ordinary skill in the art.

The grown desired bacterium may be provided to the subject in any form as long as it can grow after being administered to the subject. For example, it may be in the form of wet bacteria, dry bacteria, culture medium, or the like.

In addition, the bacterium to be provided may be provided in the form of powder, capsules, tablets, liquid such as suspension, etc., ointment, cream, or the like, though not limited to these.

### [Example]

### Storage method of bacterial flora

For storage of bacterial flora, the bacterial flora is stored at -80°C as soon as possible after collection. In the case of feces, it is collected in a collection container (feces sampling container manufactured by TechnoSuruga Laboratory Co., Ltd.) and stored in an ultra-low temperature freezer at -80°C. Alternatively, it is frozen in liquid nitrogen, and then stored in an ultra-low temperature refrigerator.

### Health condition questionnaire at the time of bacterial flora collection

An example of the donor's health condition questionnaire at the time of bacterial flora collection is shown. Questionnaire of Tables 1 and 2 is conducted on the day of the collection of bacterial flora and described therein.

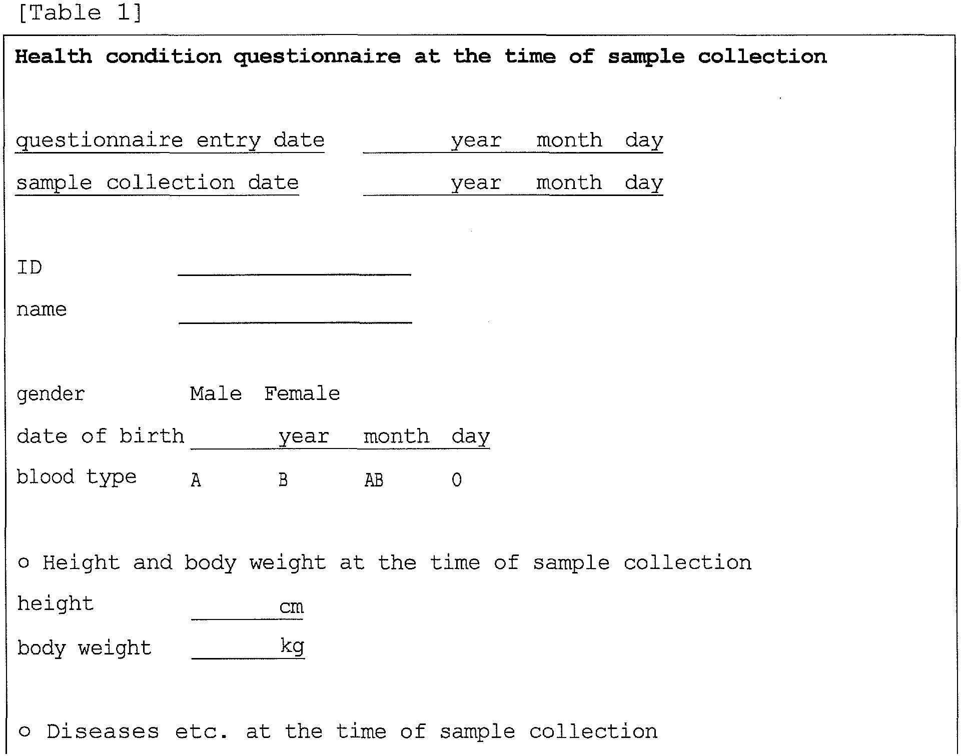

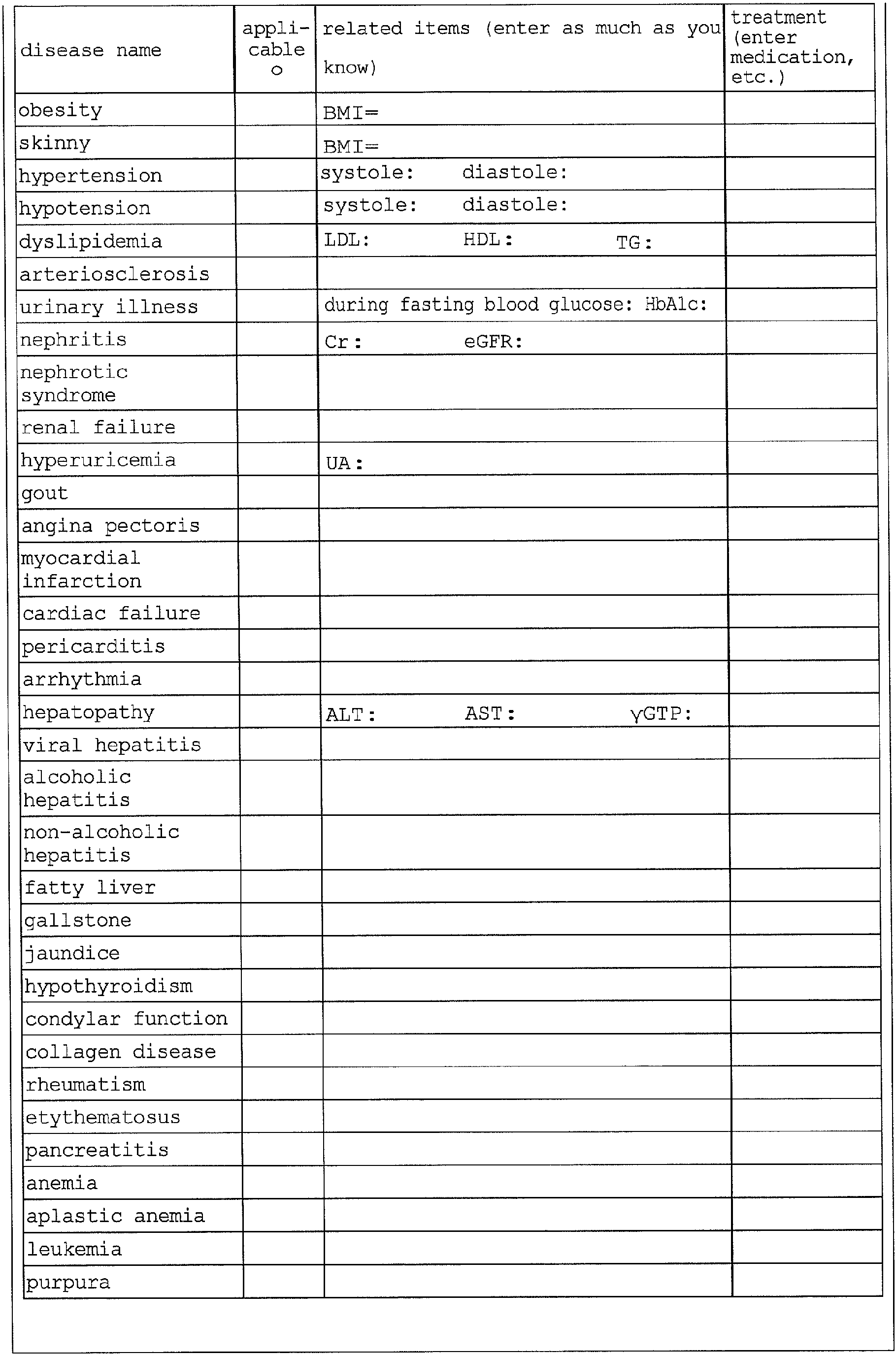

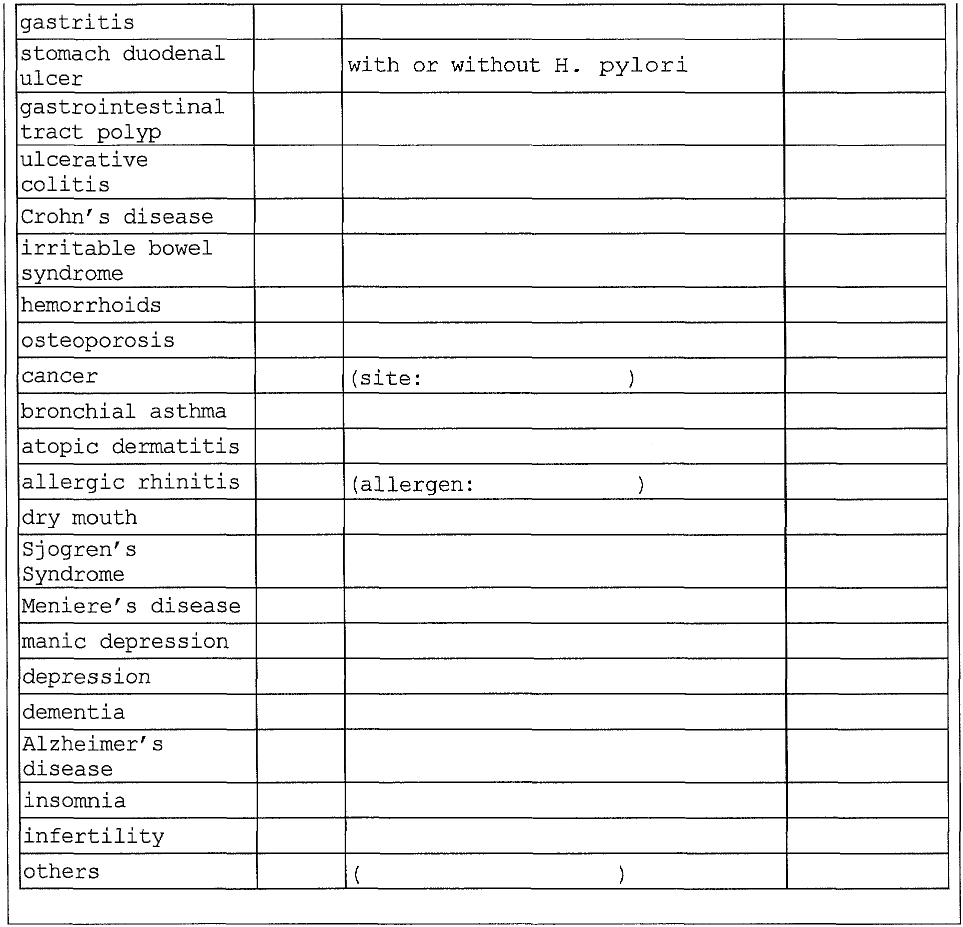

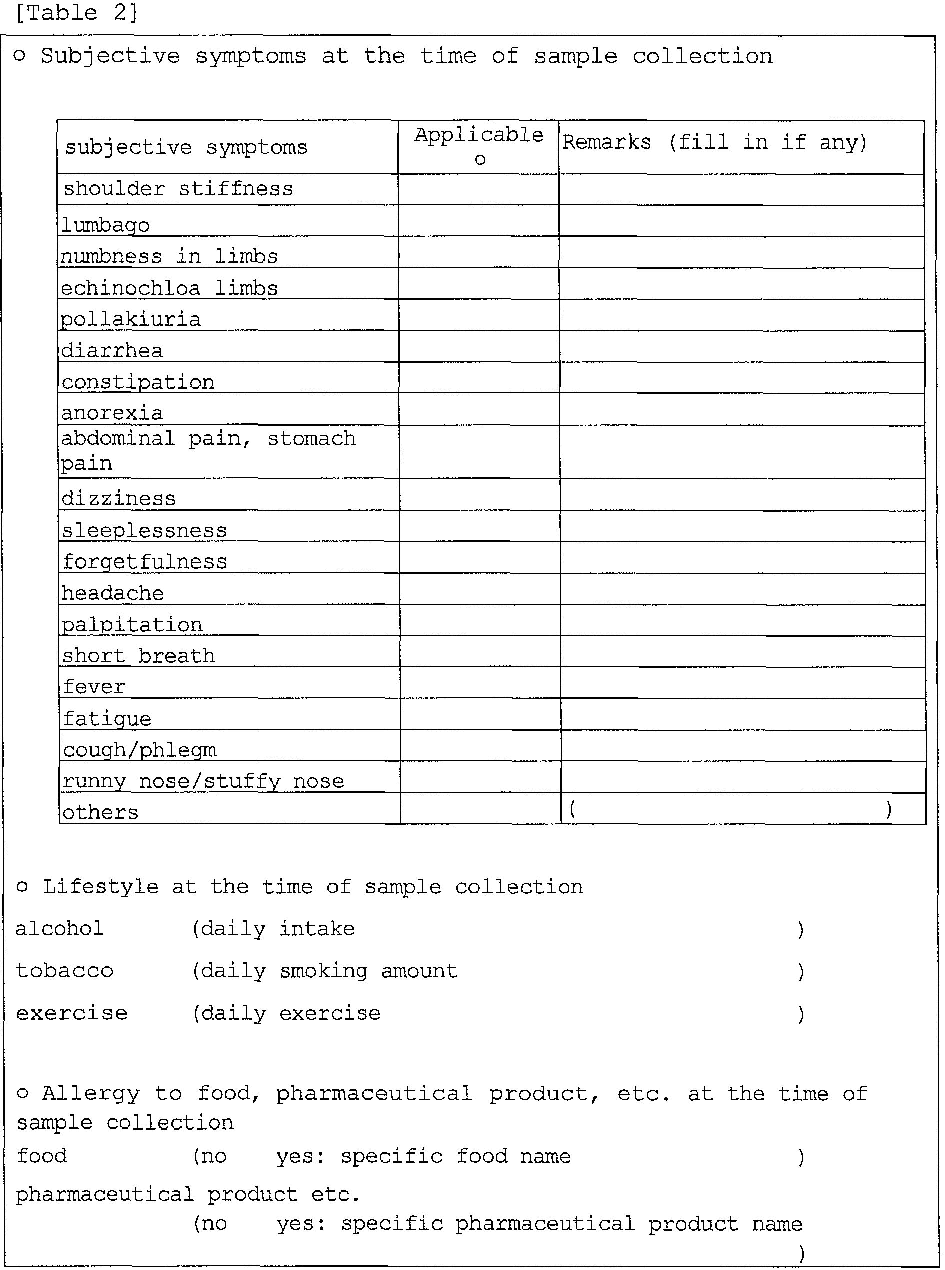

### Example of bacterial flora analysis

DNA was extracted from the collected feces (bacterial flora) by using QIAamp PowerFecal DNA kit (manufactured by QIAGEN), amplified by PCR, and then 16S metagenome analysis was performed using Miseq of Illumina Inc. An example of the result of the abundance ratio in the unit of the genus in bacterial flora is shown in Fig. 1.

### Recovery of a bacterium from bacterial flora

Recovery of bacteria was performed when Bacteroides vulgatus and Bacteroides dorei, which are closely related to arteriosclerosis, were selected from the stored bacterial flora. Feces (bacterial flora) cryopreserved at -80°C were thawed, diluted to obtain a single colony, which was plated on BL medium (Nissui Pharmaceutical Co., Ltd.) suitable for culturing Bacteroides bacteria, and was cultured at 37°C for 48 hr under anaerobic condition. From the bacteriological characteristics (morphological characteristics, etc.) of the colonies that appeared, the desired bacteria were selected and isolated. The genomic DNA was extracted from the isolated bacteria and the analysis of 16S rRNA gene was performed with the genomic DNA as the template. As a result, they were confirmed to be Bacteroides vulgatus and Bacteroides dorei.

### Culture of recovered bacterium

The desired bacterium was isolated and a large amount of the bacterium was cultured. The cells were cultured in Reinforced Clostridial Medium (Difco) at 37°C for 48 hr under anaerobic condition. The cultured bacteria were collected by centrifugation, and the collected bacteria were suspended in a Nutrient broth (Difco), placed in a vial which was then filled with nitrogen to remove oxygen.

### [Industrial Applicability]

The system of the present invention that stores bacterial flora and supplies a bacterium is a system useful for improving bacterial floras for individuals, and is a system useful for finding a bacterium that improves physical condition in personalized treatments. It is also useful for clarifying the relationship between bacterial flora and disease by constructing data. This application is based on a patent application No. 2020-129676 filed in Japan (filing date: July 30, 2020), the contents of which are incorporated in full herein.

## Claims

1. A system that supplies a bacterium to a test subject, comprising
(A) a step of constructing a database comprising data in which the changes in the health condition of donors of bacterial flora are mutually associated with the changes in the provided bacterial flora, and a bacterial flora bank in which the provided bacterial flora is stored,
(B) a step of selecting a bacterium necessary for the recovery of the health condition of a test subject based on the changes in the health condition of the test subject by using the above-mentioned database,
(C) a step of supplying the bacterium selected from the bacterial flora bank to the test subject.

2. The system according to claim 1, wherein the step (A) comprises the following steps:
(A-1) a step of analyzing the health condition of the donors of the bacterial flora and the provided bacterial flora, and constructing a database comprising data in which the changes in the health condition of the donors of the bacterial flora are mutually associated with the changes in the provided bacterial flora,
(A-2) a step of storing the provided bacterial flora and constructing a bacterial flora bank.

3. The system according to claim 2, wherein the step (A) further comprises the following step:
(A-3) a step of updating the database and the bacterial flora bank by repeating steps (A-1) and (A-2).

4. The system according to any one of claims 1 to 3, wherein the step (B) comprises the following steps:
(B-1) a step of analyzing the health condition of the test subject and the bacterial flora of the test subject, and recording data in which the changes in the health condition of the test subject are mutually associated with the changes in the bacterial flora of the test subject on the database in step (A) ,
(B-2) a step of storing the bacterial flora of the test subject in the bacterial flora bank of step (A),
(B-3) a step of searching the database for changes in the health condition of the donor which are the same as or similar to the changes in the health condition of the test subject,
(B-4) a step of confirming changes in the bacterial flora of the donor associated with the obtained changes in the health condition of the donor,
(B-5) a step of selecting a bacterium necessary for the recovery of the health condition of the test subject.

5. The system according to any one of claims 1 to 4, wherein the step (C) comprises the following steps:
(C-1) a step of isolating a bacterium selected from the bacterial flora bank,
(C-2) a step of proliferating the isolated bacterium,
(C-3) a step of supplying the proliferated bacterium to the test subject.

6. The system according to any one of claims 1 to 5, wherein the bacterial flora is a bacterial flora selected from the group consisting of intestinal bacterial flora, oral bacterial flora, skin bacterial flora and vaginal bacterial flora.

7. The system according to any one of claims 1 to 6, wherein the analysis of the bacterial flora comprises identification of bacteria contained in the bacterial flora and measurement of an abundance ratio thereof.

8. The system according to claim 7, wherein the analysis of the bacterial flora is 16s metagenome analysis or 23s metagenome analysis.

9. The system according to claim 8, comprising a sequencer for the 16s metagenome analysis or the 23s metagenome analysis.

10. The system according to any one of claims 1 to 9, wherein the health condition is a condition of infection and parasitic disease, tumor, endocrine and metabolism disease, psychiatric and nerve system disease, circulatory disease, respiratory disease, gastrointestinal disease, skin and subcutaneous tissue disease, renal genitourinary tract disease, autoimmune disease, or collagen disease.

11. The system according to any one of claims 1 to 10, comprising a non-volatile storage memory for storing database.
